Europäisches Patentamt

European Patent Office

Office européen des brevets

(19)

(11) Veröffentlichungsnummer: **0 151 470**
**B1**

(12) **EUROPÄISCHE PATENTSCHRIFT**

(45) Veröffentlichungstag der Patentschrift:
07.06.89

(21) Anmeldenummer: 85100995.1

(22) Anmeldetag: 31.01.85

(51) Int. Cl.⁴: **C 07 C 59/265**, C 07 C 51/42

(54) Verfahren zur Gewinnung von Citronensäure.

(30) Priorität: 03.02.84 DE 3403748

(43) Veröffentlichungstag der Anmeldung:
14.08.85 Patentblatt 85/33

(45) Bekanntmachung des Hinweises auf die Patenterteilung: 07.06.89 Patentblatt 89/23

(84) Benannte Vertragsstaaten:
AT BE CH DE FR GB IT LI LU NL SE

(56) Entgegenhaltungen:
DD–A– 203 533
DE–C– 1 274 128
PATENT ABSTRACTS OF JAPAN, Band 2, Nr. 136, 11.
November 1978, Seite 2927 G 78; & JP - A - 53 101 303
(KANEGAFUCHI KAGAKU KOGYO) 09-04-1978
KIRK-OTHMER ENCYCLOPEDIA OF CHEMICAL
TECHNOLOGY, Band 6, 3. Auflage, John Wiley &
Sons, New York, US

(73) Patentinhaber: Biochemie Ladenburg GmbH
D-6000 Frankfurt/Main (DE)

(72) Erfinder: Magureanu, Gabriela, Dipl.-Chem.
Delpstrasse 4
D-6940 Weinheim (DE)
Erfinder: Gutmann, Friedrich, Dipl.-Ing.
Rockenauer Pfad 25
D-6909 Walldorf (DE)

(74) Vertreter: Grussdorf, Jürgen, Dr.
Patentanwälte Zellentin & Partner Rubensstrasse 30
D-6700 Ludwigshafen (DE)

EP 0 151 470 B1

**Beschreibung**

Die vorliegende Erfindung betrifft ein neues Verfahren zur Gewinnung von Citronensäure oder anderen geeigneten Säuren und/oder ihren Salzen aus Fermentationslösungen.

Citronensäure wird z. B. als Genußsäure und Komplexbildner in der Lebensmittelindustrie, zur Metallreinigung, in der pharmazeutischen Industrie und in vielen anderen Bereichen verwendet.

Heute wird Citronensäure vor allem durch Fermentation mit Aspergillus niger aus Zuckerlösungen oder Melasse im älteren Oberflächen- oder neueren Submersverfahren gewonnen..

Je nach Art des verwendeten Ausgangsproduktes — Rübenzuckermelasse, Rohrzuckermelasse, Stärke usw. — und des verwendeten Verfahrens muß eine Vorbehandlung erfolgen, z. B. mit löslichen Hexacyanoferratsalzen, und eine Reihe von Nährsalzen mit gegebenenfalls benötigten Spurenelementen (Magnesium, Zink u. a.) muß zugeführt werden.

Abhängig von der Ausgangskonzentration an Zucker und von der Art des verwendeten Verfahrens werden so citronensäurehaltige Lösungen erhalten, die nach dem Abtrennen der Mikroorganismen neben etwa 10 bis 30 % Citronensäure noch weitere 10 bis 20 % andere organische und anorganische Stoffe enthalten. Diese Nebenprodukte stammen zu einem Teil aus der ursprünglichen Melasselösung, sind zum Teil die der Nährlösung zugesetzten und von den Mikroorganismen nicht aufgebrauchten Nährsalze und im übrigen Ausscheidungsprodukte, die der Aspergillus niger neben der Citronensäure während der Fermentation erzeugt hat.

Üblicherweise wird zur Reinigung die Citronensäure als schwer lösliches Calciumsalz ausgefällt und aus dem Calciumcitrat die Citronensäure durch Zusatz von Schwefelsäure wieder freigesetzt. Aus dieser Lösung kann die Citronensäure anschließend durch Linengen in kristalliner form gewonnen werden.

Um Citronensäure in der für Nahrungsmittel und insbesondere pharmazeutische Zwecke erforderlichen Reinheit zu gewinnen, muß dieses Verfahren allerdings wesentlich umständlicher und aufwendiger durchgeführt werden, um in jeder einzelnen Verfahrensstufe Verunreinigungen soweit wie irgend möglich abzutrennen. Einzelheiten dazu werden z. B. erläutert in Ullmann's Enzyklopädie der technischen Chemie, 3. Auflage, Bd. 5, S. 603-609 und 4. Auflage, Bd. 9, S. 631-633 sowie Kirk-Othmer Encyclopedia of Chemical Technology, 1. Auflage, Bd. 4, S. 15-17 und 2. Auflage, Bd. 5, S. 529-531. Einige Schritte seien hier genannt.

Als Nebenprodukt eventuell enthaltene Oxalsäure wird von der eigentlichen Calciumsalzfällung in saurer Lösung mit einer geringen Menge Calciumhydroxid ausgefällt. Erst nach deren Abtrennung wird Calciumcitrat bei einer Temperatur von 80 bis 95 °C durch Versetzen mit Calciumhydroxid in geringem überschuß unter Rühren kristallin ausgefällt. Das verwendete Calciumhydroxid muß dabei möglichst rein sein, da Verunreinigungen mit Magnesium, Eisen, Aluminium oder Phosphaten bei der folgenden Gewinnung der reinen Säure erheblich stören. Das erhaltene Calciumcitrat wird heiß filtriert und heiß gewaschen, um Verluste möglichst gering zu halten, da es bei 80 bis 95 °C besonders wenig löslich ist.

Das so erhaltene Calciumcitrat wird in großen Rührbehältern mit verdünnter Schwefelsäure in geringem Überschuß umgesetzt. Das ausfallende Calciumsulfat wird abfiltriert. Das braun gefärbte Filtrat bezeichnet man in der Praxis als Rohsäure. Anschließend wird diese Rohsäure z. B. mit Aktivkohle entfärbt. Die Kohle mit den darauf haftenden farbigen Verunreinigungen wird durch Filtration entfernt. Die klare Citronensäurelösung wird nach Ionenaustausch eingedampft, das Konzentrat im Vakuum oder bei Normaldruck mehrstufig kristallisiert. Je nach Reinheit müssen die rohen Kristalle noch ein- oder mehrmals unter Reinigung mit Aktivkohle umkristallisiert werden, bevor chemisch-reine Citronensäure erhalten wird. Wasserfreie Citronensäure wird durch Kristallisation oberhalb der Übergangstemperatur von 36,6 °C erhalten, darunter kristallisiert die Citronensäure als Monohydrat aus.

Neben diesem klassischen Fällungsverfahren sind in einigen Patentschriften andere Methoden beschrieben worden, nach denen die Citronensäure durch Extraktion mit organischen Lösungsmitteln gewonnen wird. Genannt seien Butylalkohol, Aceton, Tributylphosphat. Da die Kristallisation wegen der nicht vollständig entfernten Verunreinigungen sehr erschwert und die Ausbeute unbefriedigend ist, haben diese an sich eleganten Verfahren wegen Unwirtschaftlichkeit bisher in der Praxis keine Verwendung gefunden. In neuester Zeit wurde die Extraktion mit bestimmten Aminen beschrieben.

Aufgabe der Erfindung ist es, Citronensäure oder andere geeignete Säuren und/oder ihre Salze aus Fermentationslösungen durch Abtrennung der Verunreinigungen mit Hilfe spezieller physikalischchemischer Trennmethoden in einfacherer und wirtschaftlicherer Weise zu gewinnen.

Weitere Aufgabe der Erfindung ist, die mit der Citronensäureproduktion verbundene Abwasserbelastung durch aus den Rohstoffen oder dem Verfahren stammende Stoffe über deren gezielte Rückgewinnung entscheidend zu verringern.

Eine Hauptaufgabe der Erfindung ist es auch, die Citronensäureproduktion durch weitgehenden Verzicht auf die Hilfschemikalien ($Ca(OH)_2$ und $H_2SO_4$) vom sonst anfallenden Nebenprodukt $CaSO_4 \cdot 2H_2O$ zu entlasten oder ganz zu befreien, dessen Verwertung immer problematischer wird. Gleichzeitig wird das Verfahren dadurch wirtschaftlicher.

Die Lösung dieser Aufgaben, die durch die in den Ansprüchen näher gekennzeichneten Merkmale beschrieben ist, beruht auf der Abtrennung der höher molekularen Verunreinigungen durch Membranfiltration, vorzugsweise Ultrafiltration, aber auch Umkehrosmose, und anschließende Adsorption an nicht

2

ionogene Adsorptionsharze großer spezifischer Oberfläche und der Abtrennung der niedermolekularen, insbesondere anorganischen Ionen mit Hilfe von Kationen- und Anionenaustauscherharzen. Die so gereinigte Citronensäurelösung kann dann direkt kristallisiert werden oder, wie bisher, über die Calciumcitratfällung gewonnen werden.

Die Membranfiltration ist eine bekannte Methode. Für technische Ausführungen werden z. B. Hohlfaserbündel als Module zusammengefaßt und in Säulen als Batterien eingesetzt, um große Mengen von Flüssigkeit durchzusetzen. Je nach Porengröße der Membran werden so höhermolekulare Verbindungen im Konzentrat von den niedermolekularen Verbindungen im Permeat abgetrennt. Für die Reinigung von Citronensäurelösungen war diese Methode jedoch nicht einsetzbar, weil die bisher bekannten Membranen zwar Moleküle mit Molgewichten über 1000 abtrennen, aber für niedre pH-Wert von 1,5 bis 2,2 nicht beständig sind. Nunmehr sind Membranmaterialien bekannt geworden, die auch bei dieser Trennleistung solche pH-Werte über längere Zeit vertragen und bei geringem Energieaufwand ausreichend permeabel sind und sich gut reinigen lassen.

Auch diese Membranen können jedoch einen großen Teil der störenden Verunreinigungen der Fermentationslösung, d. h. solche mit Molekulargewichten von ca. 200 bis 1000 nicht abtrennen.

Die adsorptive Reinigung wäßriger und organischer Lösungen ist eine seit langem bekannte Methode. Hierbei werden organische Inhaltsstoffe an Reststoffen mit großer aktiver Oberfläche, wie Aktivkohle, Aluminiumoxid, Silikate, angelagert und angereichert. Durch Abtrennung des Adsorbens-Adsorbats-Systems nach den bekannten Verfahren der « Trennung festflüssig » können die adsorbierten Stoffe eliminiert werden. In den meisten Fällen muß in einem folgenden Verfahrensschritt das Adsorbens regeneriert werden. Solche Reinigungsverfahren kommen jedoch für gemischt organisch hochbelastete Systeme bisher wegen Verschiedenartigkeit der gelösten Verunreinigungen, ihrer unterschiedlichen Adsorbierbarkeit und nicht zuletzt aus wirtschaftlichen Gründen nur selten zur Anwendung.

Aus der deutschen Patentschrift 1 274 128 ist ein weiteres Verfahren bekannt geworden, bei dem stark heteropolare Moleküle aus wäßrigen Lösungen oder Dispersionen mit Hilfe von nicht ionogenen, wasserunlöslichen, vernetzten Polymerisaten makroretikularer Struktur abgetrennt werden können. Die Adsorptionsmittel bestehen aus Polymeren, die zu etwa 2 bis 100 % aus Vinylbenzol, Alkylvinylbenzol, Divinylbenzol oder ähnlichen monomeren Verbindungen aufgebaut sind und zusätzlich Methacrylat oder ähnliche Verbindungen sowie Äthylen, Isobutylen, Acrylnitril oder andere Monomere als Copolymerisat mit enthalten können. Verbindungen dieser Art sollen eine spezifische Oberfläche von mindestens 10 m² bis 2000 m² je g Harz aufweisen. Das Harz soll die abzutrennenden Stoffe unabhängig von einer ionischen Bindung oder irgendeinem Solvatisierungseffekt auf der äußeren und inneren Harzoberfläche durch vorwiegend van der Waals- oder Dipolkräfte binden. Als abzutrennende Stoffe werden organische Stoffe mit einer hydrophoben und hydrophilen Komponente, beispielsweise oberflächenaktive Stoffe, Reinigungsmittel, Emulgatoren, Dispersionsmittel, aber auch Steroide, wasserlösliche Enzyme, Aminosäuren, Polypeptide, Proteine und Hormone genannt. Vorteilhaft sollte das Verfahren darüber hinaus zur Abtrennung von Fettsäuren aus wäßrigen Lösungen sein. Weiterhin ist angegeben, daß auf diese Weise auch Zuckerlösungen oder Lösungen anderer chemischer Stoffe entfärbt werden können. Die adsorbierten Stoffe sollen durch Erhitzen bis zur Destillation, durch Behandlung mit Dampf zur Wasserdampfdestillation, durch Extrahieren, Auslaugen oder Desorption mit Wasser, wäßriger Säure oder alkalischen Lösungen oder mit einem organischen Lösungsmittel, welches durch fraktionierte Destillation leicht abtrennbar ist, wieder von dem Harz entfernt werden.

Erfindungsgemäß wurde festgestellt, daß auch die bei der Herstellung von Citronensäure durch Vergärung von zuckerhaltigen Rohstoffen, insbesondere bei der Submersvergärung von Rohr- oder Rübenzuckermelasse als Nebenprodukt gebildeten bzw. im Ausgangsprodukt enthaltenen Stoffe, soweit sie nicht durch die vorstehend beschriebene Membranfiltration abtrennbar sind, überwiegend an nichtionogene Polystyrol- oder Polyacrylharze, die mit 2,5 % Vinylbenzol vernetzt sind, mit einer spezifischen Oberfläche von ca. 300 bis 1000 m² pro g gebunden werden können. Die wesentlich polarere Citronensäure wird dabei nicht gebunden, so daß sie durch Nachwaschen mit reinem Wasser ohne Schwierigkeiten von dem Harz und den daran gebundenen Verunreinigungen getrennt werden kann.

Die Verunreinigungen wiederum lassen sich durch Waschen des Harzes mit wäßrigem Alkalihydroxid (pH 8 bis 13) und zusätzliches Waschen mit einem polaren organischen Lösungsmittel, wie Aceton oder niederem Alkohol, vollständig eluieren, ohne daß die Bindungskapazität des Harzes darunter leidet, so daß dieses für weitere Reinigungsoperationen wiedergewonnen werden kann.

Obwohl im Prinzip alle oben beschriebenen Harze benutzt werden können, hat sich ein Polystyrolharz als besonders günstig erwiesen, welches eine durchschnittliche Korngröße von etwa 30 US mesh (0,6 mm) und eine minimale Oberfläche von 650 m² pro g bei einer Porosität von 1,7 ml pro g besitzt. Dieses Produkt erwies sich in verschiedenen Versuchen als besonders wirkungsvoll ; Harze mit ähnlichen Eigenschaften, d. h. spezifischen Oberflächen von 500 bis 800 m²/g, Korngrößen von 0,3 bis 1 mm und Porosität von 1 bis 2 ml/g werden deshalb bevorzugt.

Bei der Durchführung des Verfahrens werden zunächst u.U. in der Fermentationslösung vorliegende echt bzw. kolloidal gelöste Hexacyanoferrate durch Zusatz einer geringen Menge von z. B. Zinkchlorid oder zinksulfat ausgefällt und mit den kolloidalen Bestandteilen sowie den höhermolekularen Verunreinigungen durch Membranfiltration mit abgeschieden. Um eine wirtschaftliche Filtration zu erzielen, wird vorzugsweise mit Membranen mit unterschiedlichem Trennschnitt gearbeitet (z. B. zunächst mit einem Trennschnitt bis zu Molekulargewichten von 50000, dann 5000 und/oder 1000 oder darunter).

Die so vorgereinigte Fermentationslösung wird dann über eine Säule mit einem Polystyrolpolymerisat, wie oben beschrieben, gegeben, bis die Verunreinigungen in größerer Menge durchbrechen, d. h. das Harz überladen ist. Je nach Harztyp kann eine Lösungsmenge, die 5 bis 15 Bett-Volumina entspricht, auf diese Art und Weise gereinigt werden.

Für den technischen Prozeß sind Säulenlängen von 1 bis 10 m geeignet, wobei längere Säulen aus Stabilitätgründen und auch um eine « quasicyclische » Verfahrensführung zu ermöglichen, in mehrere kürzere Säulen unterteilt werden können. Je nach Säulenlänge, Korngröße und Packungsdichte muß die Durchflußgeschwindigkeit auf eine möglichst gute Trennung bei noch tragbarem Durchsatz abgestimmt werden. Beladungsgeschwindigkeiten von 1 bis 10 Liter Lösung pro 1 Bett-Volumen und Stunde sind möglich, 1,5 bis 3 Liter pro Liter und Stunde haben sich als besonders günstig erweisen.

Die nach diesem Verfahren erhaltene Citronensäurelösung ist weitgehend farblos und frei von höhermolekularen Verbindungen. Anorganische Salze und eine Reihe von niedermolekularen organischen Verbindungen haben die Säule jedoch mit der Citronensäure passiert. Durch Einsatz von Ionenaustauschern können die obengenannten Substanzen eliminiert und die Citronensäure der Kristallisation zur Herstellung des Endproduktes zugeführt werden.

Die nach der Kristallisation der Citronensäure verbleibende Mutterlauge wird erneut eingesetzt, z. B. zu neuer Fermentationslösung hinzugegeben. Die Ausbeute wird dadurch weiter erhöht.

Es ist jedoch auch möglich, aus der mit Hilfe der Membranfiltration und Adsorption vorgereinigten Lösung die Citronensäure in der bekannten Weise durch Zusatz von Calciumsalzen als schwer lösliches Calciumsalz zu fällen und durch Zusatz von Schwefelsäure aus dem schwer löslichen Calciumcitrat die Citronensäure wieder freizusetzen. Durch diese Vorreinigung ist dabei das gefällte Calciumcitrat wesentlich reiner als nach dem konventionellen Verfahren, so daß einerseits der erhaltene Gips in reiner weißer Form anfällt und damit für die Weiterverarbeitung brauchbarer ist und andererseits die erhaltene Citronensäurelösung sauberer, so daß höhere Ausbeuten zu erreichen und geringere Anzahl von Kristallisationsstufen notwendig sind.

Die Membranen, deren Durchsatzleistung nach einigen Betriebsstunden abnimmt, können durch einfache Durchspülung mit beispielsweise einer 3 bis 5 prozentigen wäßrigen Hypochloritlösung gereinigt werden. Die Trennleistung wird dadurch praktisch wieder vollständig hergestellt. Um eine kontinuierliche Verfahrensweise zu ermöglichen, werden vorzugsweise mehrere Membranfiltrationssäulen parallel geschaltet, um einen Teil reinigen zu können, während die übrigen der Trennung dienen.

Die Wirtschaftlichkeit der Membranfiltration kann wesentlich verbessert werden durch eine geeignete Aufarbeitung der anfallenden Konzentrate. So können z. B. die Citronensäure durch Diafiltration oder nach Abtrennung der absetzbaren Stoffe durch erneutes Ultrafiltrieren zum größten Teil aus dem Konzentrat zurückgeführt, etwa vorhandenes Hexacyanoferrat separiert und nach entsprechender Aufarbeitung im Produktionsprozeß wieder eingesetzt sowie die enthaltenen Proteine abgetrennt und für eine eingenständige Nutzung gewonnen werden.

Besondere Bedeutung kommt der Regeneration der Polystyrolharze zu. Sinnvollerweise wird die Harzsäule zunächst mit 1 bis 2 Bett-Volumen Wasser gewaschen, um die noch enthaltene Citronensäure abzufangen. Das erste Bett-Volumen des Waschwassers hat fast die ursprüngliche Citronensäurekonzentration und wird deshalb dem Hauptdurchlauf zugesetzt, das zweite Bett-Volumen, welches nur wenig Citronensäure enthält, kann zum Waschen im nächsten Zyklus verwendet werden.

Anschließend wird die Säule vorzugsweise im Gegenstrom mit einer verdünnten wäßrigen Lauge, beispielsweise 5prozentiger Natronlauge, rückgespült, wobei 1,5 bis 2 Bett-Volumen notwendig sind, um die Hauptmenge des adsorbierten Materials zu eluieren. Die restliche organische Substanz wird durch Spülen mit 0,5 bis 1 Bett-Volumen Aceton ausgewaschen, wodurch alle Verunreinigungen praktisch quantitativ von der Säule wieder entfernt werden. Andere Lösungsmittel waren weniger gut geeignet. Die Elutionsmittel NaOH und Aceton werden durch Spülen mit Wasser aus dem System verdrängt. Die regenerierten Harze stehen somit wieder einem neuen Zyklus zur Verfügung.

Aus den Acetoneluaten wird das Lösungsmittel durch fraktionierte Destillation zum großen Teil zurückgewonnen.Die wäßrigen Phasen werden nach bekannten Abwasserreinigungsverfahren behandelt. Aus den Eluaten der Adsorptionsanlage lassen sich Humin- und Fulvinsäuren isolieren und als hochwertige Düngemittel einsetzen.

Geeignete Abwasserteilströme können auch verbrannt werden.

Die folgenden Beispiele sind für die Verfahrensführung oder Teile davon typisch, ohne daß dadurch die Erfindung jedoch eingeschränkt werden soll. Sie zeigen gleichzeitig, daß die einzelnen Reinigungsschritte in verschiedener Reihenfolge ausgeführt werden können.


Beispiel 1


Einfluß verschiedener Adsorberharze In den nachstehenden Beispielen wurden rohe, noch stark verunreinigte Citronensäurelösungen über verschiedene Amberlite-Adsorberharze und Kationenaustauscherharze gegeben.

Die genannte Lösung mit ca. 200 mval/l Citronensäure und 100 mg/l synthetischer Fulvinsäure wurde nach Vorbehandlung mit dem Kationenaustauscher Amberlite IR 120 über eine mit 50 ml unpolarem Polystyrolharz Amberlite XAD-2 gefüllte Säule bei Raumtemperatur und einer spezifischen Belastung von 4 l/l.h geleitet. Nach Beschickung mit 60 Bett-Volumina dieser Lösung wurde der Versuch abgebrochen.

Das Harz wurde anschließend mit 5 Bett-Volumina 1prozentiger NaOH-Lösung regeneriert. Dieser Zyklus wurde 10 Mal wiederholt, wobei jedesmal der Fulvinsäuregehalt über den CSB-Wert gemessen wurde.

Ergebnisse:

| Zyklus | % ads. Fulvinsäure |
|---|---|
| 1 | 76 |
| 2 | 78 |
| 3 | 70 |
| 4 | 74 |
| 5 | 82 |
| 6 | 79 |
| 7 | 78 |
| 8 | 76 |
| 9 | 74 |
| 10 | 73 |
| Mittelwert | 76 |

Eine Lösung gleicher Zusammensetzung wurde unter gleichen Bedingungen über die Adsorberharze Amberlite XAD-4, XAD-7 und XAD-1180 geführt. Es ergaben sich folgende Werte für das Rückhaltevermögen der Fulvinsäure :

| Zyklus | XAD-4 | XAD-7 | XAD-1180 |
|---|---|---|---|
| 1 | 68 | 64 | 82 |
| 2 | 70 | 60 | 84 |
| 3 | 67 | 59 | 79 |
| 4 | 69 | 62 | 80 |
| 5 | 68 | 65 | 86 |
| 6 | 72 | 60 | 80 |
| 7 | 64 | 62 | 83 |
| Mittelwert | 68 | 62 | 82 |

Beispiel 2

Rohrmelasse mit einem Zuckergehalt von 25 % wurde über 50 ml Amberlite XAD-2, XAD-4 und XAD-1180 bei Raumtemperatur und einer spezifischen Belastung von 4 l/l.h geleitet.

Nach Durchsatz von 7 Bett-Volumina wurden folgende durchschnittliche Farbaufnahme-Kapazitäten festgestellt :

| Harz-Typ | durchschnittliche Farbaufnahme in % |
|---|---|
| XAD-2 | 64 |
| XAD-4 | 50 |
| XAD-1180 | 72 |

Durch Auswaschen mit einem Bett-Volumen Aceton wurden 95 % der adsorbierten Farbstoffe entfernt. In einem vergleichbaren Versuch konnten 86 % der Farbkomponenten mit 2 Bett-Volumina Äthanol eluiert werden.

Beispiel 3

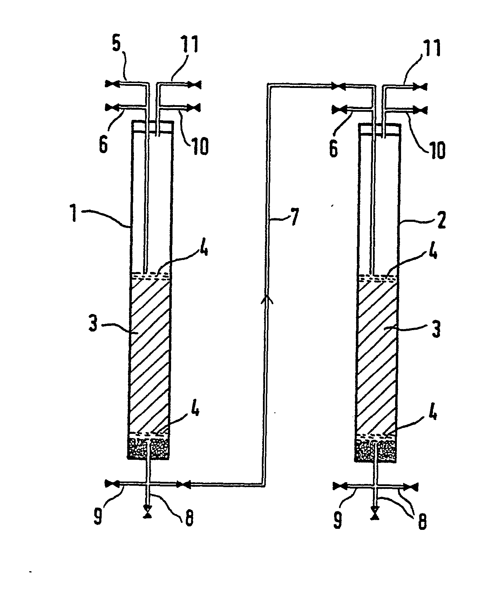

Je Zyklus werden 10 l Submersgärsäure bei einem pH-Wert von 2,0 bis 2,2 nach Zugabe von 0,25 mg Zn SO$_4$/l Gärsäure über Ultrafiltrations-Hohlfaser-Module (Romicon Inc., Niederlande) filtriert. Die Hohlfaser-Module (hollow fibre) sind selbsttragende Kapillaren, die also nur aus Membranmatrix, hier auf Polysulfonbasis, bestehen. Diese Membranfilter sind zur Abtrennung bis zu Molekulargewichten von ca. 1000 geeignet. Durch die Ultrafiltration entsteht das von Verunreinigungen verschiedener Art — wie Proteine, Cyanoferrate, gelöste höher molekulare und kolloidale Stoffe — weitgehend befreite Citronensäure-Permeat und das Konzentrat. Aus 10 l Submersgärsäure entstanden 8 l Permeat und 2 l Konzentrat. Der Eindickungsfaktor lag somit bei 5. Analysen haben ergeben, daß durch diesen Verfahrensschritt ca. 75 % der organischen Begleitkomponenten der Citronensäure entfernt wurden (als Auswertungsparameter dient der CSB). Die vorgereinigte Submersgärsäure wurde anschließend einer Adsorptionsanlge zugeführt. Die Versuchsanordnung kann der Fig. 1 entnommen werden.

In der Fig. 1 bedeuten :

  1  1. Säule
  2  2. Säule
  3  Harzbett
  4  Abdeckmaterial (z. B. Glaswolle oder Fritte)
  5  Zuleitung für Submersgärsäure
  6  Zuleitung für Regeneriermittel (Wasser)
  7  Verbindungsleitung von 1. Säule zu 2. Säule
  8  Ableitung für Eluat
  9  Zuleitung für Rückspülflüssigkeiten (Wasser, Aceton, Natronlauge)
  10 Ableitung für Rückspülflüssigkeiten
  11 Entlüftung der Säulen.

Alle Leitungen sind jeweils bis zum ersten Ventil gezeichnet und die weiterführenden Leitungen sowie die entsprechenden Vorratsbehälter, Pumpen und Vorlagen zur Vereinfachung weggelassen.

Jede Säule wurde mit 500 ml Adsorberharz XAD-1180 (Fa. Rohm & Haas, Co., USA) gefüllt. Das Harz ist ein synthetisches unlösliches Polystyrolpolymerisat, gekennzeichnet durch eine große aktive Oberflä-che (650 m$^2$/g Minimum), einen nichtionischen Charakter und eine Porosität von 1,7 ml/g Minimum.

Im nachfolgenden entsprechen die 500 ml Harz einem Bett-Volumen.

Die in Vorversuchen gewonnenen Kenntnisse haben zur folgenden optimierten Betriebsweise geführt :

| | |
|---|---|
| Spezifische Belastung | 1,5 bis 3 l/l.h |
| Regeneriertechnik | Menge (Bett-Volumen) |
| 1. Säule | 2,0 Waschwasser |
| | 1,5 NaOH (3,5 %ig) |
| | 4,0 Spülwasser |
| | 0,5 Aceton |
| | 10,0 Spülwasser |
| 2. Säule | 0,5 Aceton |
| | 10,0 Spülwasser |

Die während der Beladung und des Waschvorgangs ermittelten Werte von 4 hintereinander gefahrenen Zyklen sind in den nachstehenden Tabellen aufgezeigt.

| | |
|---|---|
| Ausgangs-Säurekonzentration : | 1,75 val/l |
| Beladung : | 1,5· l/l.h |

(Siehe Tabelle Seite 7 f.)

| Bett-vol. | 1. Zyklus | | 2. Zyklus | | 3. Zyklus | | 4. Zyklus | | 0 |
|---|---|---|---|---|---|---|---|---|---|
| | Säure val/l | Rest-farbe % | Säure val/l | Rest-farbe % | Säure val/l | Rest-farbe % | Säure val/l | Rest-farbe % | Rest-farbe % |
| 1 | – | 4,2 | 0,82 | 4,0 | – | 3,8 | – | 4,0) | ) |
| 2 | 1,72 | 9,0 | 1,70 | 9,1 | 1,70 | 8,8 | 1,70 | 9,0) | ) |
| 3 | 1,74 | 15,8 | 1,74 | 15,6 | 1,76 | 15,9 | 1,74 | 15,9)14,66) | ) |
| 4 | 1,76 | 20,4 | 1,74 | 21,4 | 1,74 | 19,3 | 1,74 | 20,4) | ) |
| 5 | 1,75 | 24,4 | 1,76 | 26,4 | 1,74 | 23,4 | 1,74 | 22,4) | )24,02 |
| 6 | 1,75 | 28,6 | 1,75 | 29,8 | 1,74 | 25,4 | 1,74 | 27,4 | ) |
| 7 | 1,78 | 31,3 | 1,74 | 31,0 | 1,74 | 29,2 | 1,75 | 32,3 | ) |
| 8 | 1,74 | 34,4 | 1,74 | 36,2 | 1,74 | 32,6 | 1,75 | 33,2 | ) |
| 9 | 1,75 | 37,5 | 1,75 | 39,3 | 1,74 | 37,6 | 1,75 | 35,6 | ) |
| 10 | 1,74 | 40,2 | 1,75 | 42,1 | 1,75 | 38,5 | 1,75 | 40,0 | ) |

Waschen: 1,5 l/l.h

| Bett-volumen | 1. Zyklus Säure val/l | 2. Zyklus Säure val/l | 3. Zyklus Säure val/l | 4. Zyklus Säure val/l |
|---|---|---|---|---|
| 1 | 1,65 | 1,42 | 1,66 | 1,68 |
| 2 | 0,30 | – | 0,33 | 0,34 |
| 3 | – | 0,024 | 0,026 | 0,030 |
| 4 | 0,009 | 0,006 | 0,008 | 0,005 |

Wie zu erwarten war, erhielt man bereits nach 2 über das Harz geleiteten Bett-Volumina Gärsäure die Ausgangs-Säurekonzentration.

Nach der Regenerierung der ersten Säule mit NaOH und anschließender Spülung wurden beide Säulen mit je 0,5 Bett-Volumen Aceton, hier also 250 ml = 196,4 g Aceton, regeneriert, das ca. 95 % der restlichen adsorbierten Stoffe entfernte. Beschickung : 5 l/l.h.

Das Aceton wird mit 10 Bett-Volumina Wasser ausgewaschen. Die folgende Tabelle gibt die Menge an, die aus Fraktionen von jeweils 1 Bett-Volumen Waschwasser in einem repräsentativen Zyklus der 1. Säule zurückgewonnen wurde.

Tabelle

| Acetonauswaschung 1. Säule | |
|---|---|
| Zugegebenes Aceton | 196,4 g |
| Bett-Volumen Waschwasser | wiedergewonnes Aceton (g) |
| 1. | 126,2 |
| 2. | 18,0 |
| 3. | 2,35 |
| 4. | 0,85 |
| 5. | 0,50 |
| 6. | 0,29 |
| 7. | 0,18 |
| 8. | 0,14 |
| 9. | 0,07 |
| 10. | 0,065 |
| wiedergewonnenes Aceton | 148,6 g |

# EP 0 151 470 B1

Diese Tabelle zeigt, daß bereits in den ersten 3 Bett-Volumina Waschwasser 98,6 % der wiedergewinnbaren Menge enthalten sind ; der Rest von 47,8 g geht während der Gegenstromregenerierung in der unvollkommen abgedichteten Apparatur bzw. bei der Wiedergewinnung verloren. Das nur noch wenig Aceton enthaltende restliche Waschwasser wird vorzugsweise beim nächsten Zyklus als Waschwasser wieder verwendet. In einer geschlossenen großtechnischen Anlage lassen sich diese Verluste vermeiden, so daß dieser Versuch insofern nicht repräsentativ ist.

Die am Adsorberharz entfärbte Gärsäure wird zur Entfernung anorganischer Salze über einen stark sauren Kationenaustauscher (Amberlite IR 120, Rohm & Haas Co. — sulfoniertes Styrol/Divinylbenzol-Copolymerisat mit 8 % DVB — und einen schwach basischen Anionenaustauscher (Amberlite IRA 68, Rohm & Haas Co. — vernetztes Acrylat mit Dialkylaminogruppen —) geleitet. Die Harze wurden in anderer Reihenfolge als in Beispiel 1 und 2 verwendet.

Die so gereinigte Gärsäure wird unter vermindertem Druck eingeengt, bis die Konzentration der Citronensäure etwa 60 g/100 g Lösung beträgt, und der Kristallisation unter vermindertem Druck bei 30 bis 35 °C unterworfen. Die mit fortschreitender Einengung (ca. 1 : 7) sich bildenden Citronensäurekristalle werden abgetrennt. Die Mutterlauge wird weiter eingeengt, bis die angereicherten Verunreinigungen eine weitere Auskristallisation verhindern.

Die Ausbeute von 3 Kristallisationsstufen betrug — unter Laborbedingungen — 89,8 % der Theorie, wobei die Kristalle der ersten Kristallisation nach einer Umkristallisation den Anforderungen der Pharmakopoe (EP Vol. III, USP 20) entsprachen.

In halbtechnischen Versuchen mit größeren Mengen an Gärsäure lag der Eindickungsfaktor bei der Ultrafiltration bei 1 : 20.

## Patentansprüche

1. Verfahren zur Gewinnung von Citronensäure oder anderen geeigneten Säuren und/oder ihren Salzen aus Lösungen, die durch Fermentation aus kohlehydrathaltigen Rohstoffen gewonnen wurden, dadurch gekennzeichnet, daß rohstoff- und/oder verfahrensbedingte höhermolekulare Verunreinigungen durch Membranfiltration, vorzugsweise Ultrafiltration, aber auch Umkehrosmose, und zusätzliche Adsorption an einem nicht ionogenen Adsorberharz mit einer spezifischen Oberfläche von 300 bis 1 000 m$^2$/g z. B. auf Polystyrol- oder Polyacrylharzbasis abgetrennt werden und die Citronensäure nach Einengen der Lösung kristallisiert wird, aus der zuvor in der bisher üblichen Weise die niedermolekularen Salze mit Hilfe von Kationen- und Anionenaustauscher gebunden wurden oder durch Fällen der Citronensäure als Calciumsalz, Umsetzen des Calciumcitrats mit Schwefelsäure, Abtrennen des Calciumsulfats und Kationen- und Anionenaustausch entfernt wurden.

2. Verfahren nach Anspruch 1, dadurch gekennzeichnet, daß Membranfiltration, Farbadsorption an Adsorberharz und Austausch der störenden Kationen und Anionen an Ionenaustauschharzen nacheinander oder in anderer geeigneter Reihenfolge durchgeführt werden, wobei einzelne Schritte auch wiederholt werden können.

3. Verfahren nach Anspruch 1 und 2, dadurch gekennzeichnet, daß der Fermentations-Lösung Zinksalze zugegeben werden und als Zinkkomplexe ausgefällte Hexacyanoferrate durch die Membranfiltration entfernt werden.

4. Verfahren nach Anspruch 1 bis 3, dadurch gekennzeichnet, daß die Gärsäure eine Gärsäure aus überwiegend Rohrzuckermelasse ist.

5. Verfahren nach einem der Ansprüche 1 bis 4, dadurch gekennzeichnet, daß die Membranen mit Hypochloritlösung oder anderen geeigneten Reinigungsmitteln gereinigt werden.

6. Verfahren nach einem der Ansprüche 1 bis 5, dadurch gekennzeichnet, daß die an das Adsorberharz gebundenen Verunreinigungen mit a) wäßrigem Alkalihydroxid pH 8 bis 13 und b) einem polaren organischen Lösungsmittel wie Aceton oder einem niederen Alkohol eluiert werden und das Harz erneut gemäß Anspruch 1 bis 5 verwendet wird.

7. Verfahren nach Anspruch 6, dadurch gekennzeichnet, daß das organische Lösungsmittel aus dem Eluat zurückgewonnen wird, z. B. durch fraktionierte Destillation.

8. Verfahren nach einem der Ansprüche 1 bis 7, dadurch gekennzeichnet, daß die Ionenaustauscher regeneriert und wiederverwendet werden.

9. Verfahren nach Anspruch 1, dadurch gekennzeichnet, daß die nach der Kristallisation der Citronensäure verbleibende Mutterlauge der Fermentationslösung zugefügt wird.

## Claims

1. Process for the obtaining of citric acid or other suitable acids and/or their salts from solutions which have been obtained by fermentation from carbohydrate-containing raw materials, characterised in that higher molecular impurities due to raw material and/or process are separated off by membrane filtration, preferably ultrafiltration, but also reverse osmosis, and additional adsorption on a non-ionic adsorber resin with a specific surface of 300 to 1 000 m$^2$/g., e. g. based on polystyrene or polyacrylate, and the citric acid is crystallised after concentration of the solution from which the lower molecular salts have previously been bound with the help of cationic or anionic exchangers or have been removed by precipitation of the citric acid as calcium salt, reaction of the calcium citrate with sulphuric acid, separation off of the calcium sulphate and cationic or anionic exchange.

2. Process according to claim 1, characterised in that membrane filtration, colour adsorption on adsorber resin and exchange of the disturbing cations and anions are carried out on ion exchanger resins successively or in other suitable sequence, whereby individual steps can also be repeated.

3. Process according to claim 1 and 2, characterised in that zinc salts are added to the fermentation solution and hexacyanoferrates precipitated out as zinc complexes are removed by membrane filtration.

4. Process according to claims 1 to 3, characterised in that the fermentation acid is a fermentation acid from preponderantly cane sugar molasses.

5. Process according to one of claims 1 to 4, characterised in that the membranes are cleaned with hypochlorite solution or other suitable cleaning agents.

6. Process according to one of claims 1 to 5, characterized in that the impurities bound to the adsorber resin are eluted with a) aqueous alkali metal hydroxide pH 8 to 13 and b) a polar organic solvent, such as acetone or a lower alcohol, and the resin is again used according to claim 1 to 5.

7. Process according to claim 6, characterised in that the organic solvent is recovered from the eluate, e. g. by fractional distillation.

8. Process according to one of claims 1 to 7, characterised in that the ion exchangers are regenerated and reused.

9. Process according to claim 1, characterised in that the mother liquor remaining after the crystallisation of the citric acid is added to the fermentation solution.

**Revendications**

1. Procédé pour obtenir l'acide citrique ou d'autres acides appropriés, et/ou des sels de ces acides, de solutions résultant de la fermentation de matières premières contenant des glucides (hydrates de carbone), procédé caractérisé en ce que l'on sépare des impuretés à haute masse moléculaire, qui proviennent de la matière première et/ou du procédé, par filtration sur membranes, de préférence par ultrafiltration, mais aussi par osmose inverse, suivie d'une adsorption sur une résine adsorbante non-ionogène ayant une surface spécifique de 300 à 1 000 m²/g, par exemple une résine de polystyrène ou une résine polyacrylique, et on fait cristalliser l'acide citrique après avoir concentré la solution de laquelle, au préalable, les sels à faible masse moléculaire ont été fixés, d'une manière jusqu'ici courante, sur des échangeurs de cations et d'anions, ou éliminés par précipitation de l'acide citrique à l'état de citrate de calcium, réaction de celui-ci avec de l'acide sulfurique, séparation du sulfate de calcium et échange de cations et anions.

2. Procédé selon la revendication 1 caractérisé en ce que l'on effectue la filtration sur membranes, l'adsorption des matières colorantes sur la résine adsorbante et l'échange des cations et anions nuisibles sur des résines d'échange d'ions, successivement dans cet ordre ou dans un autre ordre approprié, les étapes individuelles pouvant être également répétées.

3. Procédé selon la revendication 1 ou 2, caractérisé en ce que l'on ajoute des sels de zinc à la solution de fermentation et on élimine par la filtration sur membranes les hexacyanoferrates précipités à l'état de complexes de zinc.

4. Procédé selon l'une quelconque des revendications 1 à 3, caractérisé en ce que l'acide de fermentation est essentiellement un acide d'une mélasse de sucre de canne.

5. Procédé selon l'une quelconque des revendications 1 à 4, caractérisé en ce que l'on nettoye les membranes avec une solution d'hypochlorite ou avec d'autres agents de nettoyage appropriés.

6. Procédé selon l'une quelconque des revendications 1 à 5, caractérisé en ce que les impuretés qui se sont fixées sur la résine adsorbante sont éluées avec a) un hydroxyde alcalin aqueux à un pH de 8 à 13 et b) un solvant organique polaire tel que l'acétone ou un alcool inférieur, et la résine ainsi régénérée est réemployée comme dans les revendications 1 à 5.

7. Procédé selon la revendication 6 caractérisé en ce que l'on récupère le solvant organique de l'éluat, par exemple par distillation fractionnée..

8. Procédé selon l'une quelconque des revendications 1 à 7, caractérisé en ce que l'on régénère les échangeurs d'ions pour les réemployer.

9. Procédé selon la revendication 1, caractérisé en ce que l'on ajoute à la solution de fermentation la liqueur mère de la cristallisation de l'acide citrique.